(19) Europäisches Patentamt
European Patent Office
Office européen des brevets

(11) EP 1 695 692 A2

(12) # DEMANDE DE BREVET EUROPEEN

(43) Date de publication:
**30.08.2006 Bulletin 2006/35**

(51) Int Cl.:
***A61K 8/34*** *(2006.01)*

(21) Numéro de dépôt: **06100746.4**

(22) Date de dépôt: **24.01.2006**

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK YU**

(30) Priorité: **04.02.2005 FR 0550337**

(71) Demandeur: **L'Oreal-D.I.P.I.**
**75008 Paris (FR)**

(72) Inventeur: **Auguste, Frédéric**
**94550, CHEVILLY LARUE (FR)**

(74) Mandataire: **Duvert, Sandra**
**L'ORÉAL - DIPI**
**25-29 Quai Aulagnier**
**92600 Asnieres (FR)**

(54) **Composition de revêtement des fibres kératiniques comprenant une cire alcool gras et un polymère cellulosique**

(57) L'invention a pour objet une composition de revêtement des fibres kératiniques comprenant un milieu aqueux cosmétiquement acceptable caractérisée en ce qu'elle comprend au moins une cire alcool gras et au moins un polymère cellulosique.

L'invention a également pour objet l'utilisation d'une telle composition pour obtenir un maquillage chargeant des fibres kératiniques.

EP 1 695 692 A2

## Description

La présente invention a pour objet une composition cosmétique de revêtement des fibres kératiniques comprenant une cire alcool gras et un polymère cellulosique.

La composition selon l'invention peut être une composition de maquillage, encore appelée mascara, une base de maquillage des fibres kératiniques ou base-coat, une composition à appliquer sur un maquillage, dite encore top-coat, ou bien encore une composition de traitement des fibres kératiniques.
De préférence, la composition selon l'invention est une composition non rincée.

Plus spécialement, la composition selon l'invention est un mascara.
Par mascara, on entend une composition destinée à être appliquée sur les cils : il peut s'agir d'une composition de maquillage des cils, une base de maquillage des cils, une composition à appliquer sur un mascara, dite encore top-coat, ou bien encore une composition de traitement cosmétique des cils. Le mascara est plus particulièrement destiné aux cils d'êtres humains, mais également aux faux-cils.

Les compositions de maquillage pour les cils sont généralement constituées d'une cire ou mélange de cires dispersée(s) à l'aide d'au moins un tensioactif dans une phase aqueuse contenant, par ailleurs, des polymères et des pigments.
C'est généralement à travers le choix qualitatif et quantitatif des cires et polymères que sont ajustées les spécificités d'application recherchées pour les compositions de maquillage, comme par exemple leur fluidité, leur pouvoir couvrant et/ou leur pouvoir recourbant. Ainsi, il est possible de réaliser diverses compositions qui, appliquées notamment sur les cils, induisent des effets variés de type allongement, recourbement et/ou épaississement (effet chargeant).

La présente invention vise plus particulièrement à proposer une composition utile pour réaliser un maquillage épais des fibres kératiniques et notamment des cils, dit encore maquillage chargeant. Au sens de la présente invention, le terme fibres kératiniques couvre les cheveux, les cils et les sourcils et s'étend également aux postiches et faux-cils synthétiques.

Il est connu de l'art antérieur que plus la teneur en solides (apportée en partie par une phase grasse constituée par exemple, d'une ou plusieurs cires ou d'un ou plusieurs polymères lipophiles) dans une composition va augmenter, plus le dépôt de matière sur le cil va être important et donc plus le résultat obtenu sera volumateur.

Néanmoins, l'augmentation de la teneur en solides dans une composition, telle qu'une émulsion ou dispersion entraîne une augmentation de la consistance du produit obtenu et donc une application sur les cils délicate et difficile car le produit est épais, visqueux, il se dépose difficilement, de façon hétérogène et par paquets et le maquillage ainsi obtenu présente un aspect granuleux, non lisse ; le maquillage n'est pas homogène et a un aspect inesthétique.
C'est généralement le cas des mascaras dits volumateurs qui sont difficiles à appliquer et donnent un maquillage hétérogène.
Il est connu de l'art antérieur des mascaras à effet volumateur comprenant une phase aqueuse, un polymère cellulosique et des cires polaires telles que la cire de carnauba et/ou de candellila et présentant une application facile et un dépôt homogène.

La présente invention a pour but de proposer une autre voie de formulation pour une composition de révêtement des fibres kératiniques conduisant à un effet chargeant des fibres kératiniques, et qui résolve en tout ou partie les problèmes liés aux voies de formulation conventionnelles.
De manière inattendue, il a été constaté qu'il était possible de préparer des compositions permettant un maquillage épaississant des fibres kératiniques et un dépôt lisse et homogène sur lesdites fibres grâce à la mise en oeuvre, dans ces compositions, de l'association d'une cire alcool gras et d'un polymère cellulosique.

L'invention a ainsi pour objet, selon l'un de ses aspects, une composition de revêtement des fibres kératiniques comprenant un milieu aqueux cosmétiquement acceptable caractérisée en ce qu'elle comprend au 3% d'au moins une cire alcool gras et un polymère cellulosique.

Un autre objet de l'invention est une composition de revêtement des fibres kératiniques comprenant un milieu aqueux cosmétiquement acceptable caractérisée en ce qu'elle comprend au moins une cire alcool gras, au moins un polymère cellulosique et au moins un tensioactif anionique.

La présente invention vise également un procédé de maquillage des fibres kératiniques, caractérisé par le fait que l'on applique sur lesdites fibres une composition conforme à l'invention.

Elle se rapporte en outre à l'utilisation d'une composition conforme à l'invention pour obtenir un maquillage chargeant des fibres kératiniques et notamment des cils et des sourcils et/ou un dépôt lisse et homogènes sur lesdites fibres.

La présente invention a aussi pour objet l'utilisation de l'association d'au moins une cire alcool gras et d'un polymère cellulosique pour obtenir un maquillage chargeant des fibres kératiniques et notamment des cils et des sourcils et/ou un dépôt lisse et homogènes sur lesdites fibres.

Au sens de la présente invention, on entend qualifier par le terme « chargeant » la notion d'un maquillage épais et volumateur des fibres kératiniques, en particulier des cils.

Les compositions selon l'invention présentent avantageusement une teneur en matière sèche supérieure ou égale à 40 % en poids, de préférence supérieur ou égal à 42% en poids, mieux, supérieur ou égal à 45% et encore mieux supérieur ou égal à 47% par rapport au poids total de la composition, pouvant aller jusqu'à 60% en poids.

La teneur en matière sèche, c'est à dire la teneur en matière non volatile, peut être mesurée de différentes manières, on peut citer par exemple les méthodes par séchage à l'étuve, les méthodes par séchage par exposition à un rayonnement infrarouge ainsi que les méthodes chimiques par titrage de l'eau selon Karl Fischer.

De préférence, la quantité de matière sèche, communément appelée « extrait sec » des compositions selon l'invention, est mesurée par échauffement de l'échantillon par des rayons infrarouges de 2 μm à 3,5 μm de longueur d'onde. Les substances contenues dans lesdites compositions qui possèdent une pression de vapeur élevée, s'évaporent sous l'effet de ce rayonnement. La mesure de la perte de poids de l'échantillon permet de déterminer « l'extrait sec » de la composition. Ces mesures sont réalisées au moyen d'un dessiccateur à infrarouges commercial LP16 de chez Mettler. Cette technique est parfaitement décrite dans la documentation de l'appareil fournie par Mettler.

Le protocole de mesure est le suivant :

On étale environ 1g de la composition sur une coupelle métallique. Celle-ci, après introduction dans le dessiccateur, est soumise à une consigne de température de 120°C pendant une heure. La masse humide de l'échantillon, correspondant à la masse initiale et la masse sèche de l'échantillon, correspondant à la masse après exposition au rayonnement, sont mesurées au moyen d'une balance de précision.

La teneur en matière sèche est calculée de la manière suivante:

Extrait Sec = 100 x (masse sèche / masse humide).

## Cire Alcool aras

Par cire au sens de la présente invention, on entend généralement un composé lipophile, solide à température ambiante (25 °C), à changement d'état solide/liquide réversible, ayant un point de fusion supérieur ou égal à 30 °C pouvant aller jusqu'à 120 °C.

En portant la cire à l'état liquide (fusion), il est possible de la rendre miscible aux huiles et de former un mélange homogène microscopiquement, mais en ramenant la température du mélange à la température ambiante, on obtient une recristallisation de la cire dans les huiles du mélange.

En particulier, les cires convenant à l'invention peuvent présenter un point de fusion supérieur à 45° environ, mieux supérieur ou égal à 50°C et en particulier supérieur ou égal à 55 °C.

Le point de fusion de la cire peut être mesuré à l'aide d'un calorimètre à balayage différentiel (D.S.C.), par exemple le calorimètre vendu sous la dénomination MDSC 2929 par la société TA Instruments.

Le protocole de mesure est le suivant:

Un échantillon de 5 mg de produit disposé dans un creuset est soumis à une première montée en température allant de 0 °C à 120 °C, à la vitesse de chauffe de 10 °C/ minute, puis est refroidi de 120 °C à 0 °C à une vitesse de refroidissement de 10 °C/minute et enfin soumis à une deuxième montée en température allant de 0 °C à 120 °C à une vitesse de chauffe de 5 °C/minute. Pendant la deuxième montée en température, on mesure la variation de la différence de puissance absorbée par le creuset vide et par le creuset contenant l'échantillon de produit en fonction de la température.

Le point de fusion du composé est la valeur de la température correspondant au sommet du pic de la courbe représentant la variation de la différence de puissance absorbée en fonction de la température.

Les cires alcools gras peuvent être choisies parmi les alcools gras saturés ou non, ramifiés ou non, comportant de 20 à 60 atomes de carbone, ou des mélanges comprenant au moins 30% desdits alcools gras, par exemple avec du polyéthylène.

Les alcools gras peuvent être linéaires, ils peuvent comprendre par exemple de 14 à 60, de préférence de 20 à 58 atomes de carbone et correspondent de préférence à la formule chimique suivante :

CH3-(CH2)n-CH20H

dans laquelle n est un entier variant de 16 à 58, de préférence de 18 à 56.

Parmi les alcools gras ramifiés, on préférera les alcools gras comprenant de 24 à 80 atomes de carbone.

De tels alcools gras en C20-60 sont disponibles dans le commerce par exemple auprès de la société NEW PHASE TECHNOLOGIES sous les dénominations PERFORMACOL® 350, PERFORMACOL® 425, PERFORMACOL® 550 et PERFORMACOL® 700, ou auprès de la société PETROLITE sous les dénominations UNILIN® 350 Alcohol, UNILIN® 425 Alcohol, UNILIN® 550 Alcohol et UNILIN® 700 Alcohol. Il s'agit de mélanges d'alcools linéaires à très longue chaîne obtenus par un procédé de polymérisation permettant d'obtenir des polymères à très faible indice de polydispersité (Mp/Mn = 1,1). Leur masse molaire moyenne en poids est comprise entre environ 350 et 1000.

On peut également citer l'alcool béhénique, l'alcool cétylique, l'alcool stéarique et leurs mélanges.

L'alcool gras peut être présent en une teneur allant de 3 à 50% en poids, de préférence de 5 à 40%, mieux de

7 à 30% en poids et encore mieux de 10 à 30% par rapport au poids total de la composition.

Cire additionnelle

La composition selon l'invention peut comprendre au moins une cire additionnelle.

La cire additionnelle peut être choisie parmi les cires, solides et rigides à température ambiante, d'origine animale, végétale, minérale ou de synthèse et leurs mélanges.

La cire additionnelle peut également présenter une dureté allant de 0,05 MPa à 30 MPa. La dureté est déterminée par la mesure de la force en compression mesurée à 20 °C à l'aide du texturomètre vendu sous la dénomination TA-XT2 par la société RHEO, équipé d'un cylindre en inox d'un diamètre de 2 mm se déplaçant à la vitesse de mesure de 0,1 mm/s, et pénétrant dans la cire à une profondeur de pénétration de 0,3 mm.

Le protocole de mesure est le suivant :

La cire est fondue à une température égale au point de fusion de la cire + 10 °C. La cire fondue est coulée dans un récipient de 25 mm de diamètre et de 20 mm de profondeur. La cire est recristallisée à température ambiante (25 °C) pendant 24 heures de telle sorte que la surface de la cire soit plane et lisse, puis la cire est conservée pendant au moins 1 heure à 20 °C avant d'effectuer la mesure de la dureté ou du collant.

Le mobile du texturomètre est déplacé à la vitesse de 0,1 mm/s, puis pénètre dans la cire jusqu'à une profondeur de pénétration de 0,3 mm. Lorsque le mobile a pénétré dans la cire à la profondeur de 0,3 mm, le mobile est maintenu fixe pendant 1 seconde (correspondant au temps de relaxation) puis est retiré à la vitesse de 0,5 mm/s.

La valeur de la dureté est la force de compression maximale mesurée divisée par la surface du cylindre du texturomètre en contact avec la cire.

On peut notamment utiliser les cires hydrocarbonées comme la cire d'abeilles, la cire de lanoline, la cire de citron, la cire d'orange, et les cires d'insectes de Chine; la cire de riz, la cire de Carnauba, la cire de Candellila, la cire d'Ouricurry, la cire du Japon, la cire de Berry, la cire de Shellac et la cire de sumac ; la cire de montan, les cires microcristallines, les paraffines et l'ozokérite; les cires de polyéthylène, de polyméthylène, les cires obtenues par la synthèse de Fisher-Tropsch et les copolymères cireux ainsi que leurs esters.

On peut aussi citer les cires obtenues par hydrogénation catalytique d'huiles animales ou végétales ayant des chaînes grasses, linéaires ou ramifiées, en C8-C32.

Parmi celles-ci, on peut notamment citer l'huile de jojoba hydrogénée, l'huile de jojoba isomérisée telle que l'huile de jojoba partiellement hydrogénée isomérisée trans fabriquée ou commercialisée par la société Desert Whale sous la référence commerciale ISO-JOJOBA-50® , l'huile de tournesol hydrogénée, l'huile de ricin hydrogénée, l'huile de coprah hydrogénée et l'huile de lanoline hydrogénée, le tétrastéarate de di-(triméthylol-1,1,1 propane) vendu sous la dénomination « HEST 2T-4S » par la société HETERENE, le tétrabéhénate de di-(triméthylol-1,1,1 propane) vendue sous la dénomination HEST 2T-4B par la société HETERENE.

On peut encore citer les cires de silicone et les cires modifiées siliconées comme par exemple la cire de candellilla siliconée, les cires fluorées.

On peut également utiliser la cire obtenue par hydrogénation d'huile d'olive estérifiée avec l'alcool stéarylique vendue sous la dénomination « PHYTOWAX Olive 18 L 57 » ou bien encore les cires obtenues par hydrogénation d'huile de ricin estérifiée avec l'alcool cétylique vendus sous la dénomination « PHYTOWAX ricin 16L64 et 22L73 », par la société SOPHIM. De telles cires sont décrites dans la demande FR-A- 2792190.

Selon un mode de réalisation la cire présente une dureté inférieure à 4 MPa de préférence inférieure ou égale à 3,5 MPa.

De telles cires sont par exemple la cire de lanoline oxypropylénée (50E), la cire d'orange, de citron, la cire d'huile de ricin hydrogénée, le mélange d'esters d'acide aliphatiques et d'alcools primaires commercialisé sous la référence BURCO LB-02, la cire d'olive (insaponifiables d'huile d'olive hydrogénés) commercialisée sous la référence INHOLIVE par la société EXA International, la cire de béhényl méthacrylate greffé PDMS commercialisée sous la référence KP-562 par la société. SHIN ETSU, la cire de fluoropolyméthyl alkyl diméthylsiloxane telle que celle commercialisée sous la référence WAX 23087 par la société WACKER, la cire d'alkyl C30-C45 dimethicone telle que celle commercialisée sous la référence SF 1642 par GE BAYER, le tétrastéarate de ditriméthylol propane ethoxylé (50E) ou encore certaines cires de paraffine telle que la cire commercialisée sous la référence CERAFINE 56/58 par la société BAERLOCHER.

Selon un mode de réalisation particulier, la cire additionnelle est une cire dite collante c'est-à-dire possédant un collant supérieur ou égal à 0,1 N.s.

La cire collante utilisée peut posséder notamment un collant allant de 0,1 N.s à 10 N.s, en, particulier allant de 0,1 N.s à 5 N.s, de préférence allant de 0,2 à 5 N.s et mieux allant de 0,3 à 2 N.s.

Le collant de la cire est déterminé par la mesure de l'évolution de la force (force de compression) en fonction du temps, à 20°C selon le protocole indiqué précedemment pour la dureté .

Pendant le temps de relaxation de 1 s, la force (force de compression) décroît fortement jusqu'à devenir nulle puis, lors du retrait du mobile, la force (force d'étirement) devient négative pour ensuite croître à nouveau vers la valeur 0. Le

collant correspond à l'intégrale de la courbe de la force en fonction du temps pour la partie de la courbe correspondant aux valeurs négatives de la force. La valeur du collant est exprimée en N.s.

La cire collante pouvant être utilisée a généralement une dureté inférieure ou égale à 3,5 MPa, en particulier allant de 0,01 MPa à 3,5 MPa, notamment allant de 0,05 MPa à 3 MPa.
La dureté est mesurée selon le protocole décrit précédemment.

Comme cire collante, on peut utiliser un (hydroxystéaryloxy)stéarate d'alkyle en $C_{20}$-$C_{40}$ (le groupe alkyle comprenant de 20 à 40 atomes de carbone), seul ou en mélange
Une telle cire est notamment vendue sous les dénominations « Kester Wax K 82 P® » et « Kester Wax K 80 P® » par la société KOSTER KEUNEN.

La composition selon l'invention peut comprendre une teneur totale en cires (cire alcool gras et cire(s) additionnelle(s)) allant de 1 à 50% en poids par rapport au poids total de la composition, de préférence de 5 à 40 % en poids, en particulier elle peut en contenir de 10 à 35 %, plus particulièrement de 10 à 30 %.

La ou les cires (y compris la cire collante) peu(ven)t être présente(s) sous forme d'une microdispersion aqueuse de cire. On entend par microdispersion aqueuse de cire, une dispersion aqueuse de particules de cire, dans laquelle la taille desdites particules de cire est inférieure ou égale à environ 1 μm.

Les microdispersions de cire sont des dispersions stables de particules colloïdales de cire, et sont notamment décrites dans "Microemulsions Theory and Practice", L.M. Prince Ed., Academic Press (1977) pages 21-32.
En particulier, ces microdispersions de cire peuvent être obtenues par fusion de la cire en présence d'un tensioactif, et éventuellement d'une partie de l'eau, puis addition progressive d'eau chaude avec agitation. On observe la formation intermédiaire d'une émulsion du type eau-dans-huile, suivie d'une inversion de phase avec obtention finale d'une microémulsion du type huile-dans-eau. Au refroidissement, on obtient une microdispersion stable de particules colloïdales solides de cire.
Les microdispersion de cire peuvent également être obtenues par agitation du mélange de cire, de tensioactif et d'eau à l'aide de moyen d'agitation tels que les ultrasons, l'homogénéisateur haute pression, les turbines.

Les particules de la microdispersion de cire ont de préférence des dimensions moyennes inférieures à 1 μm (notamment allant de 0,02 μm à 0,99 μm), de préférence inférieures à 0,5 μm (notamment allant de 0,06 μm à 0,5 μm). Ces particules sont constituées essentiellement d'une cire ou d'un mélange de cires. Elles peuvent toutefois comprendre en proportion minoritaire des additifs gras huileux et/ou pâteux, un tensioactif et/ou un additif/actif liposoluble usuel.

Lorsque la cire ou le mélange de cires est présent, dans les compositions selon l'invention, sous la forme d'une dispersion aqueuse de particules, la taille des particules, exprimée en diamètre « effectif » moyen en volume D[4,3] tel que défini ci-après, peut être avantageusement inférieure ou égale à 1 μm et notamment inférieure ou égale à 0,75 μm. Les particules de cire peuvent présenter des formes variées. Elles peuvent notamment être sphériques.

## <u>Polymère cellulosique</u>

De préférence, le polymère cellulosique est filmogène. Par polymère "filmogène", on entend un polymère apte à former à lui seul ou en présence d'un agent auxiliaire de filmification, un film continu et adhérent sur un support, notamment sur les matières kératiniques, et de préférence un film cohésif et mieux encore, un film dont la cohésion et les propriétés mécaniques sont telles que ledit film peut être isolé dudit support.

Par polymère cellulosique, on entend la cellulose ou un dérivé de cellulose.
Le polymère cellulosique peut être choisi parmi les alkylcelluloses tels que la méthylcellulose, les hydroxyalkylcelluloses tel que l'hydroxyéthylcellulose, l'hydroxypropylcellulose l'éthylhydroxyéthylcellulose, les carboxyalkylcelluloses tels que la carboxyméthylcellulose et leurs mélanges.

Le polymère cellulosique peut être présent dans la composition selon l'invention en une teneur en matières sèches allant de 0,1 % à 30 % en poids par rapport au poids total de la composition, de préférence de 0,5 % à 20 % en poids, et mieux de 1 % à 15 % en poids.

La composition selon l'invention peut comprendre, outre le polymère cellulosique, un polymère filmogène additionnel.

Le polymère filmogène additionnel peut être présent dans la composition selon l'invention en une teneur en matières sèches allant de 0,1 % à 60 % en poids par rapport au poids total de la composition, de préférence de 0,5 % à 40 % en poids, et mieux de 1 % à 30 % en poids.

Parmi les polymères filmogènes utilisables dans la composition de la présente invention, on peut citer les polymères synthétiques, de type radicalaire ou de type polycondensat, les polymères d'origine naturelle, et leurs mélanges.

Par polymère filmogène radicalaire, on entend un polymère obtenu par polymérisation de monomères à insaturation notamment éthylénique, chaque monomère étant susceptible de s'homopolymériser (à l'inverse des polycondensats).
Les polymères filmogènes de type radicalaire peuvent être notamment des polymères, ou des copolymères, vinyliques,

notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides.

Comme monomère porteur de groupement acide, on peut utiliser des acides carboxyliques insaturés $\alpha,\beta$-éthyléniques tels que l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de monomères acides sont avantageusement choisis parmi les esters de l'acide (méth)acrylique (encore appelé les (méth)acrylates), notamment des (méth)acrylates d'alkyle, en particulier d'alkyle en $C_1$-$C_{30}$, de préférence en $C_1$-$C_{20}$, des (méth)acrylates d'aryle, en particulier d'aryle en $C_6$-$C_{10}$, des (méth)acrylates d'hydroxyalkyle, en particulier d'hydroxyalkyle en $C_2$-$C_6$.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.
Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides des monomères acides, on peut par exemple citer les (méth)acrylamides, et notamment les N-alkyl (méth)acrylamides, en particulier d'alkyl en $C_2$-$C_{12}$. Parmi les N-alkyl (méth)acrylamides, on peut citer le N-éthyl acrylamide, le N-t-butyl acrylamide, le N-t-octyl acrylamide et le N-undécylacrylamide.

Les polymères filmogènes vinyliques peuvent également résulter de l'homopolymérisation ou de la copolymérisation de monomères choisis parmi les esters vinyliqueset les monomère styréniques . En particulier, ces monomères peuvent être polymérisés avec des monomères acides et/ou leurs esters et/ou leurs amides, tels que ceux mentionnés précédemment.
Comme exemple d'esters vinyliques, on peut citer l'acétate de vinyle, le néodécanoate de vinyle, le pivalate de vinyle, le benzoate de vinyle et le t-butyl benzoate de vinyle. Comme monomère styrèniques, on peut citer le styrène, et l'alpha méthyl styrène.

Parmi les polycondensats filmogènes, on peut citer les polyuréthanes, les polyesters, les polyesters amides, les polyamides, et les résines époxyesters, les polyurées.

Les polyuréthanes peuvent être choisis parmi les polyuréthanes anioniques, cationiques, non-ioniques ou amphotères, les polyuréthanes-acryliques, les poly-uréthanes-polyvinylpirrolidones, les polyester-polyuréthanes, les polyéther-polyuréthanes, les polyurées, les polyurée-polyuréthanes, et leurs mélanges.

Les polyesters peuvent être obtenus, de façon connue, par polycondensation d'acides dicarboxyliques avec des polyols, notamment des diols.
L'acide dicarboxylique peut être aliphatique, alicyclique ou aromatique. On peut citer comme exemple de tels acides : l'acide oxalique, l'acide malonique, l'acide diméthylmalonique, l'acide succinique, l'acide glutarique, l'acide adipique, l'acide pimélique, l'acide 2,2-diméthylglutarique, l'acide azélaïque, l'acide subérique, l'acide sébacique, l'acide fumarique, l'acide maléique, l'acide itaconique, l'acide phtalique, l'acide dodécanedioïque, l'acide 1,3-cyclohexanedicarboxylique, l'acide 1,4-cyclohexanedicarboxylique, l'acide isophtalique, l'acide téréphtalique, l'acide 2,5-norbornane dicarboxylique, l'acide diglycolique, l'acide thiodipropionique, l'acide 2,5-naphtalènedicarboxylique, l'acide 2,6-naphtalènedicarboxylique. Ces monomères acide dicarboxylique peuvent être utilisés seuls ou en combinaison d'au moins deux monomères acide dicarboxylique. Parmi ces monomères, on choisit préférentiellement l'acide phtalique, l'acide isophtalique, l'acide téréphtalique.

Le diol peut être choisi parmi les diols aliphatiques, alicycliques, aromatiques. On utilise de préférence un diol choisi parmi : l'éthylène glycol, le diéthylène glycol, le triéthylène glycol, le 1,3-propanediol, le cyclohexane diméthanol, le 4-butanediol. Comme autres polyols, on peut utiliser le glycérol, le pentaérythritol, le sorbitol, le triméthylol propane.

Les polyesters amides peuvent être obtenus de manière analogue aux polyesters, par polycondensation de diacides avec des diamines ou des amino alcools. Comme diamine, on peut utiliser l'éthylènediamine, l'hexaméthylènediamine, la méta- ou para-phénylènediamine. Comme aminoalcool, on peut utiliser la monoéthanolamine.

Le polyester peut en outre comprendre au moins un monomère portant au moins un groupement $-SO_3M$, avec M représentant un atome d'hydrogène, un ion ammonium $NH_4^+$ou un ion métallique, comme par exemple un ion $Na^+$, $Li^+$, K+, $Mg^{2+}$, $Ca^{2+}$, $Cu^{2+}$, $Fe^{2+}$, $Fe^{3+}$. On peut utiliser notamment un monomère aromatique bifonctionnel comportant un tel groupement $-SO_3M$.

Le noyau aromatique du monomère aromatique bifonctionnel portant en outre un groupement $-SO_3M$ tel que décrit ci-dessus peut être choisi par exemple parmi les noyaux benzène, naphtalène, anthracène, diphényl, oxydiphényl,

sulfonyldiphényl, méthylènediphényl. On peut citer comme exemple de monomère aromatique bifonctionnel portant en outre un groupement $-SO_3M$: l'acide sulfoisophtalique, l'acide sulfotéréphtalique, l'acide sulfophtalique, l'acide 4-sulfonaphtalène-2,7-dicarboxylique.

On préfère utiliser des copolymères à base d'isophtalate/sulfoisophtalate, et plus particulièrement des copolymères obtenus par condensation de di-éthylèneglycol, cyclohexane di-méthanol, acide isophtalique, acide sulfoisophtalique.

La composition peut comprendre un polymère filmogène hydrosoluble choisi parmi :

- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique;
- les polymères d'origine naturelle, éventuellement modifiés, tels que:
    - les gommes arabiques, la gomme de guar, les dérivés du xanthane, la gomme de karaya ;
    - les alginates et les carraghénanes ;
    - les glycoaminoglycanes, l'acide hyaluronique et ses dérivés;
    - la résine shellac, la gomme de sandaraque, les dammars, les élémis, les copals ;
    - l'acide désoxyribonuciéïque ;
    - les muccopolysaccharides tels que l'acide hyaluronique, les chondroïtines sulfate, et leurs mélanges.

Selon un mode de réalisation de la composition selon l'invention, le polymère filmogène peut être un polymère solubilisé dans un milieu organique liquide de la composition comprenant des huiles ou solvants organiques tels que ceux décrits plus loin (on dit alors que le polymère filmogène est un polymère liposoluble).

A titre d'exemple de polymère liposoluble, on peut citer les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester) et d'au moins un autre monomère qui peut être un ester vinylique (différent de l'ester vinylique déjà présent), une $\alpha$-oléfine (ayant de 8 à 28 atomes de carbone), un alkylvinyléther (dont le groupe alkyl comporte de 2 à 18 atomes de carbone), ou un ester allylique ou méthallylique (ayant un radical hydrocarboné saturé, linéaire ou ramifié, de 1 à 19 atomes de carbone, lié au carbonyle du groupe ester).

Ces copolymères peuvent être réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères : acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.

Comme polymères filmogènes liposolubles, on peut également citer les copolymères liposolubles, et en particulier ceux résultant de copolymérisation d'esters vinyliques ayant de 9 à 22 atomes de carbone ou d'acrylates ou de méthacrylates d'alkyle, les radicaux alkyles ayant de 10 à 20 atomes de carbone.

De tels copolymères liposolubles peuvent être choisis parmi les copolymères de polystéarate de vinyle, de polystéarate de vinyle réticulé à l'aide de divinylbenzène, de diallyléther ou de phtalate de diallyle, les copolymères de poly(méth)acrylate de stéaryle, de polylaurate de vinyle, de poly(méth)acrylate de lauryle, ces poly(méth)acrylates pouvant être réticulés à l'aide de diméthacrylate de l'éthylène glycol ou de tétraéthylène glycol.

Les copolymères liposolubles définis précédemment sont connus et notamment décrits dans la demande FR-A-2232303 ; ils peuvent avoir un poids moléculaire moyen en poids allant de 2.000 à 500.000 et de préférence de 4.000 à 200.000.

Comme polymères filmogènes liposolubles utilisables dans l'invention, on peut également citer les polyalkylènes et notamment les copolymères d'alcènes en $C_2$-$C_{20}$, comme le polybutène, les alkylcelluloses avec un radical alkyle linéaire ou ramifié, saturé ou non en $C_1$ à $C_8$ comme l'éthylcellulose et la propylcellulose, les copolymères de la

vinylpyrolidone (VP) et notamment les copolymères de la vinylpyrrolidone et d'alcène en $C_2$ à $C_{40}$ et mieux en $C_3$ à $C_{20}$. A titre d'exemple de copolymère de VP utilisable dans l'invention, on peut citer le copolymère de VP/acétate vinyle, VP/méthacrylate d'éthyle, la polyvinylpyrolidone (PVP) butylée, VP/méthacrylate d'éthyle/acide méthacrylique, VP/eicosène, VP/hexadécène, VP/triacontène, VP/styrène, VP/acide acrylique/méthacrylate de lauryle.

Le polymère filmogène peut être également présent dans la composition sous la forme de particules en dispersion dans une phase aqueuse ou dans une phase solvant non aqueuse (milieu organique liquide de la composition), connue généralement sous le nom de latex ou pseudolatex. Les techniques de préparation de ces dispersions sont bien connues de l'homme du métier.

Comme dispersion aqueuse de polymère filmogène, on peut utiliser les dispersions acryliques vendues sous les dénominations Neocryl XK-90® , Neocryl A-1070® , Neocryl A-1090® , Neocryl BT-62® , Neocryl A-1079® et Neocryl A-523® par la société AVECIA-NEORESINS, Dow Latex 432® par la société DOW CHEMICAL, Daitosol 5000 AD® ou Daitosol 5000 SJ® par la société DAITO KASEY KOGYO; Syntran 5760® par la société Interpolymer ou encore les dispersions aqueuses de polyuréthane vendues sous les dénominations Neorez R-981® et Neorez R-974® par la société AVECIA-NEORESINS, les Avalure UR-405® , Avalure UR-410® , Avalure UR-425® , Avalure UR-450® , Sancure 875® , Sancure 861® , Sancure 878® et Sancure 2060® par la société GOODRICH, Impranil 85® par la société BAYER, Aquamere H-1511® par la société HYDROMER ; les sulfopolyesters vendus sous le nom de marque Eastman AQ® par la société Eastman Chemical Products, les dispersions vinyliques comme le Mexomère PAM® de la société CHIMEX et leurs mélanges.

La composition selon l'invention peut comprendre un agent plastifiant favorisant la formation d'un film avec le polymère filmogène. Un tel agent plastifiant peut être choisi parmi tous les composés connus de l'homme du métier comme étant susceptibles de remplir la fonction recherchée.

**Milieu aqueux cosmétiquement acceptable**

Le milieu aqueux cosmétiquement acceptable de la composition selon l'invention peut être constituée essentiellement d'eau ; il peut également comprendre un mélange d'eau et de solvant miscible à l'eau (miscibilité dans l'eau supérieure à 50 % en poids à 25 °C) comme les monoalcools inférieurs ayant de 1 à 5 atomes de carbone tels que l'éthanol, l'isopropanol, les glycols ayant de 2 à 8 atomes de carbone tels que le propylène glycol, l'éthylène glycol, le 1,3-butylène glycol, le dipropylène glycol, les cétones en $C_3$-$C_4$, les aldéhydes en $C_2$-$C_4$ et leur mélanges.

Le milieu aqueux (eau et éventuellement le solvant miscible à l'eau) peut être présent en une teneur allant de 0,1 % à 95 % en poids, par rapport au poids total de la composition, de préférence allant de 1 % à 80 % en poids

Système émulsionnant

Selon l'invention, on utilise un système émulsionnant choisi de manière appropriée pour l'obtention d'une émulsion huile-dans-eau ou cire-dans-eau.

Les compositions selon l'invention peuvent contenir des agents tensioactifs émulsionnants présents notamment en une proportion allant de 0,1 à 40 %, et mieux de 0,3 % à 20 % en poids par rapport au poids total de la composition.

Les tensioactifs ioniques mis en oeuvre dans le cadre de la présente invention peuvent être anioniques, cationiques ou non ionique ou amphotériques. Toutefois, généralement, le choix d'au moins un tensioactif anionique est privilégié.

A titre représentatif des tensioactifs anioniques convenant à l'invention, on peut plus particulièrement citer :

- les sels d'acides gras en $C_{16}$-$C_{30}$ notamment ceux dérivant des amines, comme le stéarate de triéthanolamine et/ou le stéarate d'amino-2 méthyl-2 propane di-ol-1,3;
- les sels d'acides gras polyoxyéthylénés notamment ceux dérivant des amines ou les sels alcalins, et leurs mélanges ;
- les esters phosphoriques et leurs sels tels que le "DEA oleth-10 phosphate" (Crodafos N 10N de la société CRODA) ;
- les sulfosuccinates tels que le "Disodium PEG-5 citrate lauryl sulfosuccinate" et le "Disodium ricinoleamido MEA sulfosuccinate"
- les alkyléthersulfates tels que le lauryl éther sulfate de sodium;
- les iséthionates ;
- les acylglutamates tels que le "Disodium hydrogenated tallow glutamate" (AMISOFT HS-21 R commercialisé par la société AJINOMOTO) et leurs mélanges.

Conviennent tout particulièrement à l'invention, le stéarate de triéthanolamine et/ou le stéarate d'amino-2 méthyl-2 propane di-ol-1,3. Ce dernier est généralement obtenu par simple mélange de l'acide stéarique avec de la triéthanolamine et/ou de l'amino-2 méthyl-2 propane di-ol-1,3.

D'une manière générale, les compositions selon l'invention peuvent contenir de 0,1 à 30 % en poids, en particulier de

0,5 à 20 % en poids voire de 1 à 15 % en poids de tensioactif anionique par rapport au poids total de la composition.

A titre représentatif des tensioactifs cationiques, on peut notamment citer:

- les alkyl-imidazolidinium tels que l'étho-sulfate d'isostéaryl-éthylimidonium,
- les sels d'ammonium tels que le chlorure de N,N,N-triméthyl-1-docosanaminium (chlorure de Behentrimonium).

La composition selon l'invention peut en outre comprendre au moins agent tensioactif non ionique ou amphotérique. On peut se reporter au document « Encyclopedia of Chemical Technology, KIRK-OTHMER », volume 22, p. 333-432, 3ème édition, 1979, WILEY, pour la définition des propriétés et des fonctions (émulsionnant) des tensioactifs, en particulier p. 347-377 de cette référence, pour les tensioactifs anioniques, amphotériques et non ioniques.

## Phase grasse

Par "phase grasse", on entend, au sens de l'invention, une phase composée d'un ou plusieurs corps non aqueux liquides ou solides à température ambiante (25°C), généralement compatibles entre eux, tels que les cires, les corps gras pâteux, les huiles, les huiles épaissies par un agent structurant et leurs mélanges. Les tensioactifs tels que décrit plus haut ne font pas partie de la phase grasse.

Ainsi, la phase grasse de la composition selon l'invention comprend au moins une cire alcool gras et éventuellement une cire additionnelle telles que décrites plus haut.

La phase grasse présente avantageusement une dureté inférieure ou égale à 6 MPa, par exemple allant de 0,5 à 6 MPa, de préférence de 0,7 à 4 MPa et mieux de 0,8 à 3 MPa.

La phase grasse peut représenter de 5% à 60% en poids par rapport au poids total de la composition, de préférence de 10% à 50% et mieux de 15% à 40% en poids.

### *Huiles*

La composition selon l'invention peut notamment comprendre une ou plusieurs huiles.

L'huile peut être choisie parmi les huiles volatiles et/ou les huile non volatiles, et leurs mélanges. La composition comprend avantageusement au moins une huile volatile.

L'huile peut être présente dans la composition selon l'invention en une teneur allant de 0,1 % à 60 % en poids, de préférence de 0,1 % à 30 % en poids par rapport au poids total de la composition.

Par " huile volatile", on entend au sens de l'invention une huile susceptible de s'évaporer au contact de la peau ou de la fibre kératinique en moins d'une heure, à température ambiante et pression atmosphérique. Le ou les solvants organiques volatils et les huiles volatiles de l'invention sont des solvants organiques et des huiles cosmétiques volatiles, liquides à température ambiante, ayant une pression de vapeur non nulle, à température ambiante et pression atmosphérique, allant en particulier de 0,13 Pa à 40 000 Pa ($10^{-3}$ à 300 mm de Hg), en particulier allant de 1,3 Pa à 13 000 Pa (0,01 à 100 mm de Hg), et plus particulièrement allant de de 1 ,3 Pa à 1300 Pa (0,01 à 10 mm de Hg).

Par "huile non volatile", on entend une huile restant sur la peau ou la fibre kératinique à température ambiante et pression atmosphérique au moins plusieurs heures et ayant notamment une pression de vapeur inférieure à $10^{-3}$ mm de Hg (0,13 Pa).

Ces huiles peuvent être des huiles hydrocarbonées, des huiles siliconées, des huiles fluorées, ou leurs mélanges.

On entend par "huile hydrocarbonée", une huile contenant principalement des atomes d'hydrogène et de carbone et éventuellement des atomes d'oxygène, d'azote, de soufre, de phosphore. Les huiles hydrocarbonées volatiles peuvent être choisies parmi les huiles hydrocarbonées ayant de 8 à 16 atomes de carbones, et notamment les alcanes ramifiés en C8-C16 comme les isoalcanes en C8-C16 d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars' ou de Permetyls, les esters ramifiés en C8-C16 le néopentanoate d'iso-hexyle, et leurs mélanges. D'autres huiles hydrocarbonées volatiles comme les distillats de pétrole, notamment ceux vendus sous la dénomination Shell Solt par la société SHELL, peuvent aussi être utilisées. De préférence, le solvant volatil est choisi parmi les huiles volatiles hydrocarbonées ayant de 8 à 16 atomes de carbone et leurs mélanges.

Comme huiles volatiles, on peut aussi utiliser les silicones volatiles, comme par exemple les huiles de silicones linéaires ou cycliques volatiles, notamment celles ayant une viscosité ≤ 8 centistokes ($8\ 10^{-6}\ m^2/s$), et ayant notamment de 2 à 7 atomes de silicium, ces silicones comportant éventuellement des groupes alkyle ou alkoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

On peut également citer les huiles linéaires alkyltrisiloxanes volatiles de formule générale (I)

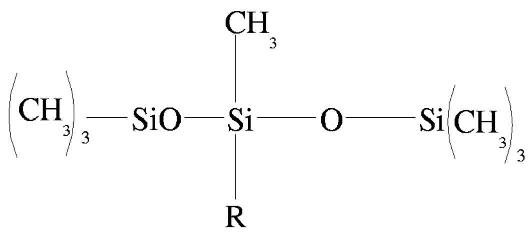

où R représente un groupe alkyle comprenant de 2 à 4 atomes de carbone et dont un ou plusieurs atomes d'hydrogène peuvent être substitués par un atome de fluor ou de chlore.
Parmi les huiles de formule générale (I), on peut citer :

le 3-butyl 1,1,1,3,5,5,5-heptaméthyl trisiloxane,
le 3-propyl 1,1,1,3,5,5,5-heptaméthyl trisiloxane, et
le 3-éthyl 1,1,1,3,5,5,5-heptaméthyl trisiloxane,
correspondant aux huiles de formule (I) pour lesquelles R est respectivement un groupe butyle, un groupe propyle ou un groupe éthyle.

On peut également utiliser des solvants volatils fluorés tels que le nonafluorométhoxybutane ou le perfluorométhylcyclopentane.
On peut également utiliser des solvants volatils fluorés tels que le nonafluorométhoxybutane ou le perfluorométhylcyclopentane.
La composition peut également comprendre au moins une huile non volatile, et notamment choisie parmi les huiles hydrocarbonées et/ou siliconées et/ou fluorées non volatiles.
Comme huile hydrocarbonée non volatile, on peut notamment citer :

- les huiles hydrocarbonées d'origine végétale telles que les triesters d'acides gras et de glycérol dont les acides gras peuvent avoir des longueurs de chaînes variées de C4 à C24, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore les triglycérides des acides caprylique/caprique comme ceux vendus par la société Stéarineries Dubois ou ceux vendus sous les dénominations Miglyol 810, 812 et 818 par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane, et leurs mélanges;
- les esters de synthèse comme les huiles de formule R1 COOR2 dans laquelle R1 représente le reste d'un acide gras linéaire ou ramifié comportant de 1 à 40 atomes de carbone et R2 représente une chaîne hydrocarbonée notamment ramifiée contenant de 1 à 40 atomes de carbone à condition que R5 + R6 soit ≥ 10, comme par exemple l'huile de Purcellin (octanoate de cétostéaryle), le myristate d'isopropyle, le palmitate d'isopropyle, le benzoate d'alcool en C12 à C15, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de diisostéaryle ; et les esters du pentaérythritol ;
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, le 2-undécylpentadécanol ;
- les acides gras supérieurs tels que l'acide oléique, l'acide linoléique, l'acide linolénique ;
- les carbonates,
- les acétales,
- les citrates,
- et leurs mélanges.

Les huiles de silicone non volatiles utilisables dans la composition selon l'invention peuvent être les polydiméthylsiloxanes (PDMS) non volatiles, les polydiméthylsiloxanes comportant des groupements alkyle ou alcoxy, pendant

et/ou en bout de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates ;
Les huiles fluorées utilisables dans l'invention sont notamment des huiles fluorosiliconées, des polyéthers fluorés, des silicones fluorées telles que décrit dans le document EP-A-847752.

***Agent structurant***

L'agent structurant de(s) l'huile(s), si présent, peut être choisi parmi les polymères semi-cristallin, les gélifiants lipophiles et leur mélanges.

On entend par polymère des composés comportant au moins deux motifs, de préférence au moins 3 motifs et plus spécialement au moins 10 motifs de répétition. Par "polymère semi-cristallin", on entend des polymères comportant une partie cristallisable, une chaîne pendante cristallisable ou une séquence cristallisable dans le squelette, et une partie amorphe dans le squelette et présentant une température de changement de phase réversible du premier ordre, en particulier de fusion (transition solide-liquide). Lorsque la partie cristallisable est sous forme d'une séquence cristallisable du squelette polymérique, la partie amorphe du polymère est sous forme de séquence amorphe ; le polymère semi-cristallin est dans ce cas un copolymère séquencé par exemple du type dibloc, tribloc ou multibloc, comportant au moins une séquence cristallisable et au moins une séquence amorphe. Par "séquence", on entend généralement au moins 5 motifs de répétition identiques. La ou les séquences cristallisables sont alors de nature chimique différente de la ou des séquences amorphes.
Le polymère semi-cristallin a une température de fusion supérieure ou égale à 30°C (notamment allant de 30°C à 80 °C), de préférence allant de 30°C à 60 °C. Cette température de fusion est une température de changement d'état du premier ordre.
Cette température de fusion peut être mesurée par toute méthode connue et en particulier à l'aide d'un calorimètre à balayage différentiel (D.S.C).
De façon avantageuse, le ou les polymères semi-cristallins auxquelles s'applique l'invention présentent une masse moléculaire moyenne en nombre supérieure ou égale à 1 000. De façon avantageuse, le ou les polymères semi-cristallins de la composition de l'invention ont une masse moléculaire moyenne en nombre Mn allant de 2 000 à 800 000, de préférence de 3 000 à 500 000, mieux de 4 000 à 150 000, notamment inférieure à 100 000, et mieux de 4 000 à 99 000. De préférence, ils présentent une masse moléculaire moyenne en nombre supérieure à 5 600, allant par exemple de 5 700 à 99 000. Par "chaîne ou séquence cristallisable", on entend au sens de l'invention une chaîne ou séquence qui si elle était seule passerait de l'état amorphe à l'état cristallin, de façon réversible, selon qu'on est au-dessus ou en dessous de la température de fusion. Une chaîne au sens de l'invention est un groupement d'atomes, pendant ou latéral par rapport au squelette du polymère. Une séquence est un groupement d'atomes appartenant au squelette, groupement constituant un des motifs répétitif du polymère. Avantageusement, la "chaîne pendante cristallisable" peut être chaîne comportant au moins 6 atomes de carbone.
Le polymère semi-cristallin peut être choisi parmi les copolymères séquencés comportant au moins une séquence cristallisable et au moins une séquence amorphe, les homopolymères et les copolymères portant au moins une chaîne latérale cristallisable par motif de répétition, leurs mélanges.
De tels polymères sont décrits par exemple dans le document EP 1396259.
Selon un mode plus particulier de réalisation de l'invention, le polymère est issu d'un monomère à chaîne cristallisable choisi parmi les (méth)acrylates d'alkyle saturés en $C_{14}$ à $C_{22}$.
A titre d'exemple particulier de polymère semi-cristallin structurant utilisable dans la composition selon l'invention, on peut citer les produits Intelimer® de la société Landec décrits dans la brochure "Intelimer® polymers", Landec IP22 (Rev. 4-97). Ces polymères sont sous forme solide à température ambiante (25°C). Ils sont porteurs de chaînes latérales cristallisables.

Les gélifiants utilisables dans les compositions selon l'invention peuvent être des gélifiants lipophiles organiques ou minéraux, polymériques ou moléculaires.
Comme gélifiant lipophile minéral, on peut citer les argiles éventuellement modifiées comme les hectorites modifiées par un chlorure d'ammonium d'acide gras en $C_{10}$ à $C_{22}$, comme l'hectorite modifiée par du chlorure de di-stéaryl diméthyl ammonium telle que, par exemple, celle commercialisée sous la dénomination de Bentone 38V® par la société ELEMENTIS.
On peut également citer la silice pyrogénée éventuellement traitée hydrophobe en surface dont la taille des particules est inférieure à 1 µm. Il est en effet possible de modifier chimiquement la surface de la silice, par réaction chimique générant une diminution du nombre de groupes silanol présents à la surface de la silice. On peut notamment substituer des groupes silanol par des groupements hydrophobes: on obtient alors une silice hydrophobe. Les groupements hydrophobes peuvent être :

- des groupements triméthylsiloxyle, qui sont notamment obtenus par traitement de silice pyrogénée en présence de l'hexaméthyldisilazane. Des silices ainsi traitées sont dénommées « Silica silylate » selon le CTFA (6ème édition, 1995). Elles sont par exemple commercialisées sous les références Aerosil R812® par la société DEGUSSA, CAB-O-SIL TS-530® par la société CABOT,
- des groupements diméthylsilyloxyle ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références Aerosil R972® , et Aerosil R974® par la société DEGUSSA, CAB-O-SIL TS-610® et CAB-O-SIL TS-720® par la société CABOT.

La silice pyrogénée hydrophobe présente en particulier une taille de particules pouvant être nanométrique à micrométrique, par exemple allant d'environ de 5 à 200 nm.

Les gélifiants lipophiles organiques polymériques sont par exemple les organopolysiloxanes élastomériques partiellement ou totalement réticulés, de structure tridimensionnelle, comme ceux commercialisés sous les dénominations de KSG6® , KSG16® et de KSG18® par la société SHIN-ETSU, de Trefil E-505C® et Trefil E-506C® par la société DOW-CORNING, de Gransil SR-CYC® , SR DMF10® , SR-DC556® , SR 5CYC gel® , SR DMF 10 gel® et de SR DC 556 gel® par la société GRANT INDUSTRIES, de SF 1204® et de JK 113® par la société GENERAL ELECTRIC ; l'éthylcellulose comme celle vendue sous la dénomination Ethocel® par la société DOW CHEMICAL ; les polycondensats de type polyamide résultant de la condensation entre ($\alpha$) au moins un acide choisi parmi les acides dicarboxyliques comprenant au moins 32 atomes de carbone tels que les acides gras dimères et ($\beta$) un alkylène diamine et en particulier l'éthylène diamine, dans lequel le polymère polyamide comprend au moins un groupe acide carboxylique terminal estérifié ou amidifié avec au moins un mono alcool ou une mono amine comprenant de 12 à 30 atomes de carbone linéaires et saturés, et en particulier, les copolymères d'éthylène diamine/dilinoléate de stéaryle tel que celui commercialisé sous la dénomination Uniclear 100 VG® par la société ARIZONA CHEMICAL ; les galactommananes comportant de un à six, et en particulier de deux à quatre, groupes hydroxyle par ose, substitués par une chaîne alkyle saturée ou non, comme la gomme de guar alkylée par des chaînes alkyle en $C_1$ à $C_6$, et en particulier en $C_1$ à $C_3$ et leurs mélanges. Les copolymères séquencés de type "dibloc", "tribloc" ou "radial" du type polystyrène/polyisoprène, polystyrène/polybutadiène tels que ceux commercialisés sous la dénomination Luvitol HSB® par la société BASF, du type polystyrène/copoly(éthylène-propylène) tels que ceux commercialisés sous la dénomination de Kraton® par la société SHELL CHEMICAL CO ou encore du type polystyrène/copoly(éthylène-butylène), les mélanges de copolymères tribloc et radial (en étoile) dans l'isododécane tels que ceux commercialisé par la société PENRECO sous la dénomination Versagel® comme par exemple le mélange de copolymère tribloc butylène/éthylène/styrène et de copolymère étoile éthylène/propylène/styrène dans l'isododécane (Versagel M 5960).

On peut également utiliser les polyamides siliconés du type polyorganosiloxane tels que ceux décrits dans les documents US-A-5 874 069, US-A-5,919,441, US-A-6,051,216 et US-A-5,981,680.

Ces polymères siliconés peuvent appartenir aux deux familles suivantes:

- des polyorganosiloxanes comportant au moins deux groupes capables d'établir des interactions hydrogène, ces deux groupes étant situés dans la chaîne du polymère, et/ou
- des polyorganosiloxanes comportant au moins deux groupes capables d'établir des interactions hydrogène, ces deux groupes étant situés sur des greffons ou ramifications.

Parmi les gélifiants lipophiles pouvant être utilisés dans les compositions selon l'invention, on peut encore citer les esters de dextrine et d'acide gras, tels que les palmitates de dextrine, notamment tels que ceux commercialisés sous les dénominations Rheopearl TL® ou Rheopearl KL® par la société CHIBA FLOUR.

**Additifs**

La composition selon l'invention peut également comprendre une matière colorante comme les matières colorantes pulvérulentes, les colorants liposolubles, les colorants hydrosolubles. Cette matière colorante peut être présente en une teneur allant de 0,01 % à 30 % en poids, par rapport au poids total de la composition.

Les matières colorantes pulvérulentes peuvent être choisies parmi les pigments et les nacres.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium, de zinc ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les nacres peuvent être choisies parmi les pigments nacrés blancs tels que le mica recouvert de titane ou d'oxychlorure de bismuth, les pigments nacrés colorés tels que le mica titane avec des oxydes de fer, le mica titane

avec notamment du bleu ferrique ou de l'oxyde de chrome, le mica titane avec un pigment organique du type précité ainsi que les pigments nacrés à base d'oxychlorure de bismuth.

Les colorants liposolubles sont par exemple le rouge Soudan, le D&C Red 17, le D&C Green 6, le β-carotène, l'huile de soja, le brun Soudan, le D&C Yellow 11, le D&C Violet 2, le D&C orange 5, le jaune quinoléine, le rocou. Les colorants hydrosolubles sont par exemple le jus de betterave, le bleu de méthylène, le sel disodique de ponceau, le sel disodique du vert d'alizarine, le jaune de quinoléine, le sel trisodique d'amarante, le sel disodique de tartrazine, le sel monosodique de rhodamine, le sel disodique de fuchsine, la xanthophylle.

La composition selon l'invention peut également comprendre une charges choisie parmi celles bien connues de l'homme du métier et couramment utilisées dans les compositions cosmétiques. Les charges peuvent être minérales ou organiques, lamellaires ou sphériques. On peut citer le talc, le mica, la silice, le kaolin, les poudres de polyamide comme le Nylon® (Orgasol de chez Atochem), de poly-β-alanine et de polyéthylène, les poudres de polymères de tétrafluoroéthylène comme le Téflon® , la lauroyl-lysine, l'amidon, le nitrure de bore, les micro sphères creuses polymériques expansées telles que celles de chlorure de polyvinylidène/acrylonitrile comme l'ExpanceL® (Nobel Industrie), les poudres acryliques telles que le polytrap® (Dow Corning), les particules de polyméthacrylate de méthyle et les microbilles de résine de silicone (Tospearls® de Toshiba, par exemple), le carbonate de calcium précipité, le carbonate et l'hydro-carbonate de magnésium, l'hydroxyapatite, les microsphères de silice creuses (SILICA BEADS® de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les charges peuvent représenter de 0,1 à 25 %, et mieux de 1 à 20 % en poids du poids total de la composition.

La composition de l'invention peut comprendre, en outre, tout additif usuellement utilisé en cosmétique tels que les antioxydants, les conservateurs, les parfums, les neutralisants, les gélifiants hydrophiles, les épaississants, les vitamines, les fibres et leurs mélanges.

Bien entendu l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

Les gélifiants hydrophiles utilisables dans les compositions selon l'invention peuvent être choisi parmi :

- les homo- ou copolymères d'acides acrylique ou méthacrylique ou leurs sels et leurs esters et en particulier les produits vendus sous les dénominations VERSICOL F® ou VERSICOL K® par la société ALLIED COLLOID, UTRAHOLD 8® par la société CIBA-GEIGY, les acides polyacryliques de type SYNTHALEN K,
- les copolymères d'acide acrylique et d'acrylamide vendus sous la forme de leur sel de sodium sous les dénominations RETEN® par la société HERCULES, le polyméthacrylate de sodium vendu sous la dénomination DARVAN N°7® par la société VANDERBILT, les sels de sodium d'acides polyhydroxycarboxyliques vendus sous la dénomination HYDAGEN F® par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- les copolymères AMPS/acrylamide de type SEPIGEL® ou SIMULGEL® commercialisés par la société SEPPIC, et
- les copolymères AMPS/méthacrylates d'alkyle polyoxyéthylénés (réticulés ou non), et leurs mélanges.

Les polymères filmogènes hydrosolubles cités plus haut peuvent également jouer le rôle de gélifiant hydrosoluble.

Le polymère gélifiant hydrosoluble peut être présent dans la composition selon l'invention en une teneur en matières sèches allant de 0,01 % à 60 % en poids, de préférence de 0,5 % à 40 % en poids, mieux de 1 % à 30 % en poids, voire de 5 à 20 % en poids par rapport au poids total de la composition.

Par "fibre", il faut comprendre un objet de longueur L et de diamètre D tel que L soit très supérieur à D, D étant le diamètre du cercle dans lequel s'inscrit la section de la fibre. En particulier, le rapport L/D (ou facteur de forme) est choisi dans la gamme allant de 3,5 à 2500, de préférence de 5 à 500, et mieux de 5 à 150.

En particulier, les fibres ont une longueur allant de 1 μm à 10 mm, de préférence de 0,1 mm à 5 mm et mieux de 0,3 mm à 3mm .

Les fibres utilisables dans la composition de l'invention peuvent être choisies parmi les fibres rigides ou non rigides, elles peuvent être d'origine synthétique ou naturelle, minérale ou organique.

A titre de fibres utilisables dans la composition selon l'invention, on peut citer les fibres non rigides telles que les fibres de polyamide (Nylon® ) ou les fibres rigides telles que les fibres de polyimide-amide comme celles vendues sous les dénomination "KERMEL", " KERMEL TECH" par la société RHODIA ou de poly-(p-phénylène-téréphtalamide) (ou d'aramide) notamment vendues sous la dénomination Kevlar® par la société DUPONT DE NEMOURS.

De préférence, la composition selon l'invention est un mascara.

Les compositions selon l'invention peuvent être préparées selon des méthodes connues de l'homme du métier.

La composition selon l'invention peut être conditionnée dans un récipient délimitant au moins un compartiment qui comprend ladite composition, ledit récipient étant fermé par un élément de fermeture.

Le récipient est de préférence associé à un applicateur, notamment sous forme d'une brosse comportant un arrangement de poils maintenus par un fil torsadé. Une telle brosse torsadée est décrite notamment dans le brevet US 4 887 622. Il peut être également sous forme d'un peigne comportant une pluralité d'éléments d'application, obtenus notamment de moulage. De tels peignes sont décrits par exemple dans le brevet FR 2 796 529. L'applicateur peut être solidaire du récipient, tel que décrit par exemple le brevet FR 2 761 959. Avantageusement, l'applicateur est solidaire d'une tige qui, elle même, est solidaire de l'élément de fermeture.

L'élément de fermeture peut être couplé au récipient par vissage. Alternativement, le couplage entre l'élément de fermeture et le récipient se fait autrement que par vissage, notamment via un mécanisme à baïonnette, par encliquetage, ou par serrage. Par "encliquetage" on entend en particulier tout système impliquant le franchissement d'un bourrelet ou d'un cordon de matière par déformation élastique d'une portion, notamment de l'élément de fermeture, puis par retour en position non contrainte élastiquement de ladite portion après le franchissement du bourrelet ou du cordon.

Le récipient peut être au moins pour partie réalisé en matériau thermoplastique. A titre d'exemples de matériaux thermoplastiques, on peut citer le polypropylène ou le polyéthylène.

Alternativement, le récipient est réalisé en matériau non thermoplastique, notamment en verre ou en métal (ou alliage).

Le récipient est de préférence équipé d'un essoreur disposé au voisinage de l'ouverture du récipient. Un tel essoreur permet d'essuyer l'applicateur et éventuellement, la tige dont il peut être solidaire. Un tel essoreur est décrit par exemple dans le brevet FR 2 792 618.

Le contenu des brevets ou demandes de brevets cités précédemment sont incorporés par référence dans la présente demande.

Les exemples qui suivent sont présentés à titre illustratif et non limitatif de l'invention.
Les quantités indiquées sont en pourcentage pondéral et exprimées par rapport au poids total de la composition, sauf instructions contraires.

**Exemple 1 : Mascara**

On peut préparer le mascara suivant:

| | |
|---|---|
| Hydroxyéthyl cellulose | 1 |
| triéthanolamine | 3,1 |
| acide stéarique | 5,8 |
| Cire alcool (Performacol 350 de NEW PHASE TECHNOLOGIES) | 30 |
| Eau | Qsp100 |

**Exemple 2 à 4 : Mascaras**

On prépare 2 mascaras (exemples 3 et 4) selon l'invention comprenant une cire alcool gras (alcool béhénique) et un polymère cellulosique et un mascara selon l'art antérieur (exemple 2) comprenant un polymère cellulosique mais pas de cire alcool gras.

| | Exemple 2 (hors invention) | Exemple 3 | Exemple 4 |
|---|---|---|---|
| hydroxyéthyl cellulose | 1 | 1 | 1 |
| Triéthanolamine | 3,1 | 3,1 | 3,1 |
| acide stéarique | 5,8 | 5,8 | 5,8 |
| alcool béhénique | 0 | 10 | 17 |
| Cire collante (Kester Wax K82P de Koster Keunen) | 20 | 10 | 3 |
| Eau | Qsp 100 | Qsp 100 | Qsp 100 |

Pour chaque exemple, on mesure la charge in vitro.
La charge in vitro est mesurée par gravimétrie sur 2 éprouvettes de faux cils qui sont des cheveux caucasiens courbes (15 cheveux longs d'environ 15 mm répartis sur une distance de 1 cm).

Les faux cils sont maquillés par le dessous en réalisant 3x10 passages de mascara espacés de 2 minutes avec reprise de produit entre chaque série de 10.

Les 2 éprouvettes sont séchées 10 min à température ambiante puis pesées.

On détermine la charge à To ($C_{To}$) de l'éprouvette avant séchage qui correspond à la charge mesurée après séchage ($C_{sec}$) divisée par l'extrait sec de la composition (ES).

$$C_{To} = C_{sec} / ES$$

On obtient les résultats suivants

| | Charge $C_{To}$ (en g) |
|---|---|
| **Exemple 2** | 0,0025 |
| **Exemple 3** | 0,0031 |
| **Exemple 4** | 0,0042 |

Les compositions des exemples 3 et 4 selon l'invention présentent une charge plus élevée que la composition ne comprenant pas de cire alcool gras. On constate également que la charge augmente avec la teneur en cire alcool gras.

**Revendications**

1. Composition de revêtement des fibres kératiniques comprenant un milieu aqueux cosmétiquement acceptable **caractérisée en ce qu'**elle comprend au moins 3 % d'au moins une cire alcool gras et au moins un polymère cellulosique.

2. Composition de revêtement des fibres kératiniques comprenant un milieu aqueux cosmétiquement acceptable **caractérisée en ce qu'**elle comprend au moins une cire alcool gras, au moins un polymère cellulosique et au moins un tensioactif anionique.

3. Composition selon la revendication 1 ou 2, **caractérisée en ce que** le polymère cellulosique est choisis parmi les alkylcelluloses, les hydroxyalkylcelluloses, les carboxyalkylcelluloses et leurs mélanges.

4. Composition selon l'une des revendications 1 à 3, **caractérisée en ce que** le polymère cellulosique est présent en une teneur en matières sèches allant de 0,1 % à 30 % en poids par rapport au poids total de la composition, de préférence de 0,5 % à 20 % en poids, et mieux de 1 % à 15 % en poids.

5. Composition selon l'une quelconque des revendications précédentes, **caractérisée en ce que** la cire alcool gras est choisie parmi les alcools gras saturés ou non, ramifiés ou non, comportant de 14 à 80 atomes de carbone, ou des mélanges comprenant au moins 30% desdits d'alcools gras.

6. Composition selon l'une quelconque des revendications précédentes, **caractérisée en ce que** la cire alcool gras est choisie parmi les alcools gras linéaires comprenant de 14 à 60 atomes de carbone, de préférence de 20 à 58 atomes de carbone, les alcools ramifiés comprenant de 24 à 80 atomes de carbone et leurs mélanges.

7. Composition selon l'une quelconque des revendications précédentes, **caractérisée en ce que** la cire alcool gras représente de 3 à 50% en poids, de préférence de 5 à 40%, mieux de 7 à 30% en poids et encore mieux de 10 à 30% en poids par rapport au poids total de la composition.

8. Composition selon l'une des revendications précédentes, **caractérisée en ce qu'**elle comprend au moins une cire additionnelle.

9. Composition selon la revendication précédente, **caractérisée en ce que** la cire additionnelle est choisie parmi les cires hydrocarbonées comme la cire d'abeilles, la cire de lanoline, la cire de citron, la cire d'orange et les cires d'insectes de Chine; la cire de riz, la cire de Carnauba, la cire de Candellila, la cire d'Ouricurry, la cire de fibres de

liège, la cire de canne à sucre, la cire du Japon, la cire de Berry, la cire de Shellac et la cire de sumac; la cire de montan, les cires microcristallines, les paraffines, l'ozokérite; les cires de polyéthylène, de polyméthylène, les cires obtenues par la synthèse de Fisher-Tropsch, les copolymères cireux ainsi que leurs esters, les cires obtenues par hydrogénation catalytique d'huiles animales ou végétales ayant des chaînes grasses, linéaires ou ramifiées, en C8-C32, les cires de silicone, les cires fluorées et leurs mélanges.

**10.** Composition selon la revendication 8 ou 9, **caractérisée en ce que** la cire additionnelle présente une dureté inférieure ou égale à 4 MPa, de préférence inférieure ou égale à 3,5 MPa.

**11.** Composition selon l'une quelconque des revendications 8 à 10, **caractérisée en ce que** la cire additionnelle est choisie parmi les cires présentant un collant supérieur ou égal à 0,1 N.s.

**12.** Composition selon l'une quelconque des revendications 8 à 11, **caractérisée par le fait que** la cire additionnelle est un (hydroxystéaroyloxy)stéarate d'alkyle en $C_{20}$-$C_{40}$.

**13.** Composition selon l'une quelconque des revendications 8 à 12, **caractérisée par le fait que** la cire additionnelle est présente en une teneur allant de 5 à 50% en poids par rapport au poids total de la composition, de préférence de 5 à 40 % en poids, plus particulièrement de 10 à 35% en poids.

**14.** Composition selon l'une quelconque des revendications 1 et 3 à 13, **caractérisée en ce qu'**elle comprend au moins un tensioactif anionique.

**15.** Composition selon la revendication 2 ou 14, **caractérisée en ce que** ledit tensioactif anionique est choisi parmi les sels d'acides gras en $C_{16}$-$C_{30}$ ; les sels d'acides gras polyoxyéthoxylénés ; les esters phosphoriques et leurs sels ; les alkyléthersulfates ; les sulfosuccinates ; les iséthionates et les acylglutamates et leurs mélanges.

**16.** Composition selon la revendication 14 ou 15, **caractérisée en ce que** le tensioactif anionique comprend au moins le stéarate de triéthanolamine et/ou le stéarate d'amino-2 méthyl-2 propane di-ol-1,3.

**17.** Composition selon l'une quelconque des revendications 14 à 16, **caractérisée en ce que** le tensioactif anionique est présent à raison de 0,01 à 30 % en poids et notamment de 0,1 à 15 % en poids du poids total de la composition.

**18.** Composition l'une quelconque des revendications précédentes, **caractérisée en ce qu'**elle comprend un polymère filmogène additionnel choisi parmi les polymères synthétiques, de type radicalaire ou de type polycondensat, les polymères d'origine naturelle et leurs mélanges.

**19.** Composition selon la revendication 18, **caractérisée en ce que** le polymère filmogène additionnel est présent en une teneur en matières sèches allant de 0,1 % à 60 % en poids par rapport au poids total de la composition, de préférence de 0,5 % à 40 % en poids, et mieux de 1 % à 30 % en poids.

**20.** Composition selon l'une quelconque des revendications précédentes, **caractérisée en ce que** milieu aqueux est présent en une teneur allant de 0,1 % à 95 % en poids, par rapport au poids total de la composition, de préférence allant de 1 % à 80 % en poids.

**21.** Composition selon l'une des revendications précédentes, **caractérisée en ce qu'**elle comprend une matière colorante.

**22.** Composition selon la revendication 21, **caractérisée en ce que** la matière colorante est présente en une teneur allant de 0,01 % à 30 % en poids, par rapport au poids total de la composition.

**23.** Composition selon l'une des revendications 1 à 22, **caractérisée en ce qu'**elle est un mascara.

**24.** Utilisation d'une composition selon l'une des revendications 1 à 23, pour obtenir un maquillage chargeant des fibres kératiniques et notamment des cils et/ou un dépôt lisse et homogènes sur lesdites fibres.

**25.** Utilisation de l'association d'au moins une cire alcool gras et d'un polymère cellulosique pour obtenir un maquillage chargeant des fibres kératiniques et notamment des cils et des sourcils et/ou un dépôt lisse et homogènes sur lesdites fibres.

**26.** Procédé cosmétique de traitement ou de maquillage des fibres kératiniques comprenant l'application sur les dites fibres kératiniques d'une composition selon l'une quelconque des revendications 1 à 23.